# EUROPEAN PATENT SPECIFICATION

(11) **EP 2 529 666 B1**
(45) Date of publication and mention of the grant of the patent: **18.01.2017**
(21) Application number: 11736907.4
(22) Date of filing: 20.01.2011
(51) Int. Cl.: A61B 8/08

(54) **ULTRASONIC DIAGNOSIS DEVICE AND METHOD USED THEREFOR TO TRACK MEASUREMENT POINT**
ULTRASCHALLDIAGNOSEVORRICHTUNG SOWIE DARIN VERWENDETES VERFAHREN ZUR VERFOLGUNG VON MESSSTELLEN
DISPOSITIF DE DIAGNOSTIC ULTRASONIQUE ET PROCÉDÉ L'UTILISANT POUR SUIVRE UN POINT DE MESURE

(30) Priority: 29.01.2010 JP 2010019124
(43) Date of publication of application: 05.12.2012
(73) Proprietor: Hitachi, Ltd., Chiyoda-ku, Tokyo 100-8280 (JP)
(72) Inventor: CHONO,Tomoaki, Tokyo 101-0021 (JP)
(74) Representative: MERH-IP Matias Erny Reichl Hoffmann Patentanwälte PartG mbB
(86) International application number: PCT/JP2011/050912
(87) International publication number: WO 2011/093193

(56) References cited:
- JP-A- 2004 208 807
- JP-A- 2008 142 568
- JP-A- 2008 289 873
- JP-A- 2010 000 199
- US-A- 5 615 680
- US-A1- 2002 072 674
- US-A1- 2008 317 316
- US-A1- 2009 131 788
- US-A1- 2009 318 803

## Description

### Field of the Invention

The present invention relates to an ultrasonic diagnostic apparatus, in particular to a technique for tracking the movement of biological tissue on the basis of an ultrasonic image of biological tissue of an object to be examined, which calculates and displays a specific physical quantity correlated with the movement or property of biological tissue based on the tracking results.

### Description of Related Art

An ultrasonic diagnostic apparatus transmits ultrasonic waves to the inside of an object via an ultrasonic probe and receives the reflected echo signals of ultrasonic waves in conformity to the structure of the biological tissue from the inside of the object, so as to construct an ultrasonic image (for example, an ultrasonic tomographic image such as a B-mode image) and displays the image for the use of diagnosis.

In recent years, a technique has been used for diagnosis which tracks the movement of biological tissue on the basis of an ultrasonic image, and calculates a specific physical quantity correlated with the movement or property of the biological tissue (hereinafter referred to merely as physical quantity) on the basis of the tracking result. For example, when a target for diagnosis is cardiac muscle, a technique is known which calculates and displays a specific physical quantity such as the moving velocity of cardiac muscle or the strain (distortion) which is the property of cardiac muscle tissue on the basis of the tracking result, for diagnosis of a heart disease such as ischemic heart disease.

As for the method of tracking biological tissue in ultrasonic diagnostics, techniques such as tissue Doppler and speckle tracking have been proposed. Speckle tracking in particular, is capable of tracking the positions where biological tissue has been moved and quantifying the distortion of a region in a living body which is related to the biological tissue without depending on the direction of ultrasonic beams, and is applied, for example to track the movement of the cardiac muscle of an object.

As for the method of tracking the movement of cardiac muscle, as disclosed in Patent Document 1, a technique is known which executes a tracking process by extracting a plurality of traceable points from an ultrasonic image, and calculates a specific physical quantity on the basis of the movement information on the tracked points. Also, as disclosed in Non-patent Document 1, a technique is known which measures cardiac muscle of the left ventricle by segmenting it into 17 regions and makes a diagnosis of a disease based on the respective measurement values.

### Prior Art Documents

### Patent Document

Patent Document 1: Japanese Patent No. 4060615

### Non-patent Document

Non-patent Document 1: Robert M. Lang, et al., Recommendations for Chamber Quantification, Journal of American Society of Echocardiography, Vo. 18, No. 12

However, the conventional techniques disclosed in the above-mentioned Patent Document 1 and Non-patent Document 1 do not consider the measurement of expansion and contraction of cardiac muscle. Effective reflection of expansion and contraction of cardiac muscle facilitates the measurement values of cardiac muscle to be a target for comparison to make appropriate diagnosis.

In the method of tracking the movement of cardiac muscle, it is common to set a region of interest in the cardiac muscle on an ultrasonic image and track the cardiac muscle in a plurality of measurement points (tracking points) in the region of interest. In this regard, the conventional techniques do not consider the setting a plurality of measurement points along the expansion/contraction direction of cardiac muscle (for example, the direction along the endocardium and the epicardium of cardiac muscle).

Therefore, for example, in the case that the variation of distance between the adjacent measurement points caused by expansion/contraction of cardiac muscle, the expansion and contraction of the cardiac muscle may not be accurately reflected to the respective measurement points. Also, since the distance from the respective measurement points to the endocardium or the epicardium of the cardiac muscle is not constant, even when the variation of the distance caused by expansion and contraction of the cardiac muscle is measured, the respective measurement values may not be suitable reference for comparison to make a proper diagnosis.

US 2002/072674 A1 describes a method for performing strain rate analysis for ultrasonic images in which the spatial gradient of velocity is calculated in the direction of tissue motion. Strain rate is calculated for cardiac ultrasound images in the direction of motion which, for myocardial images, may be either in the plane of the myocardium or across the myocardium. Strain rate information is calculated for a sequence of images of a heart cycle and displayed for an automatically drawn border such as the endocardial border over the full heart cycle.

US 2008/317316 A1 describes a method comprising the steps: Obtaining the position of each of points composing the contour of a specific tissue shown in ultrasonic image data having been acquired at each time phase by pattern matching for each time phase; obtaining motion information of each of parts composing the specific tissue based on the position of each of the points composing the contour; for each time phase, obtaining the differential value of the motion information of each of the parts by differentiating the motion information of each of the parts by time, and normalizing the differential value of the motion information; assigning a color corresponding to the magnitude of the normalized differential value of the motion information to each of the parts; displaying an ultrasonic image at each time phase and furthermore displaying each of the parts of the specific tissue shown in the ultrasonic image of each time phase in the assign color.

US 2009/318803 A1 describes a method where a plurality of strain gauges defined by gauge endpoints are set in each time phase using motion vector information of tissue, and a three-dimensional strain gauge image in which each strain gauge is disposed at a three-dimensional position corresponding to, for example, an ultrasonic image in each time phase is generated and displayed. Moreover, an MPR image is set on volume data and is displayed in a predetermined form in a state where gauge coordinates are projected thereon.

US 2009/131788 A1 describes an ultrasonic imaging system and method for quantification and display of myocardial wall thickening. The endocardial and epicardial borders in an image sequence are defined over a heart cycle and changes in the distance between the borders are tracked at specified locations around the myocardium over the heart cycle, The changes in distance are presented to the user in a graphical format, preferably together with another measure of the cardiac cycle such as chamber volume variation, ejection fraction, or the ECG waveform. The changes in the distance of chord lengths across the myocardium provide a direct indication of wall thickness variation at the specified locations. Preferably the tracking of the specified locations over the heart cycle is done by speckle tracking. The inventive technique can also represent strain at the specified locations of the myocardium.

US 5 615 680 A describes a diagnostic ultrasound system comprising an element for detecting a velocity of motion of a tissue, such as a cardiac muscle or a vascular wall, contained in an object at every sampling point in a section of the object and an element for creating two-dimensionally mapped data of the velocity in the section. The system further comprises an element for analyzing a motion state of the tissue on the basis of local velocities falling into a plurality of local regions set on the two-dimensionally mapped data of the velocity and an element for displaying analysis results of the motion state. The velocity detecting element transmits an ultrasonic pulse signal toward the tissue in order to acquire a Doppler shifted echo, according to a pulsed Doppler technique.

The objective of the present invention is to provide the ultrasonic diagnostic apparatus and the measurement-point tracking method capable of measuring expansion and contraction of the cardiac muscle, which facilitates accurate reflection of expansion and contraction of cardiac muscle to the respective measurement points for making a proper diagnosis.

### Brief Summary of the Invention

In order to achieve the above-described objective, in the present invention, a measurement position setting unit sets a region of interest in the cardiac muscle on an ultrasonic image which is displayed on a display unit, and a tracking calculation unit tracks a plurality of measurement points by a plurality of first segmenting lines along the epicardium and the endocardium of the cardiac muscle in a plurality of measurement points of the region of interest and a plurality of second segmenting lines that are orthogonal to the plurality of first segmenting lines.

In concrete terms, the ultrasonic diagnostic apparatus of the present invention comprises:
an ultrasonic probe configured to transmit/receive ultrasonic waves to/from an object;
an ultrasonic signal generating unit configured to generate ultrasonic signals on the basis of the reflected echo signals, that are received by the ultrasonic probe, of the cross section of the tissue including the cardiac muscle of the heart of the object;
an ultrasonic image generating unit configured to generate an ultrasonic image on the basis of the ultrasonic signals;
a display unit configured to display the ultrasonic image;
a measurement position setting unit configured to set a region of interest in the cardiac muscle on an ultrasonic image which is displayed on the display unit;
a tracking calculation unit configured to track the movement of cardiac muscle in a plurality of meshed pattern measurement points of the region of interest; and
a physical quantity calculating unit configured to calculate a specific physical quantity based on the tracking result,
wherein:
the calculated specific physical quantity is displayed on the display unit; and
the tracing calculation unit tracks the plurality of meshed pattern measurement points by a plurality of segmenting lines along the epicardium and the endocardium of the cardiac muscle and a plurality of second segmenting lines that are orthogonal to the plurality of first segmenting lines, wherein the plurality of measurement points are set on every intersections of the first segmenting lines and the second segmenting lines (223), and wherein the physical quantity calculating unit calculates both a radial strain and a circumference strain by using every two adjacent measurement points.

Also, the present invention relates to a measurement point tracking method as defined in independent claim 8.

### Effect of the Invention

In accordance with the present invention, it is possible to measure expansion and contraction of cardiac muscle which facilitates accurate reflection of expansion and contraction of cardiac muscle to the respective measurement values for making a proper diagnosis.

### Brief Description of the Drawings

Fig. 1 is a block diagram showing the general configuration of the ultrasonic diagnostic apparatus in the present embodiment.
Fig. 2 is a flowchart showing the processing flow of the ultrasonic diagnostic apparatus in the present embodiment.
Fig. 3 is an example of a display screen in the ultrasonic diagnostic apparatus.
Fig. 4 is an example of a display screen showing measurement results.
Fig. 5 is another example of a display screen in the ultrasonic diagnostic apparatus.
Fig. 6 is an enlarged view of a region of interest.
Fig. 7 is an example of the measurement method in a 3-dimensional ultrasonic image.

### Detailed Description of the Invention

An embodiment of the ultrasonic diagnostic apparatus to which the present invention is applied will be described below. In the following description, the same function parts are represented by the same reference numerals, and the duplicative description thereof is omitted.

### (Configuration of Ultrasonic Diagnostic Apparatus)

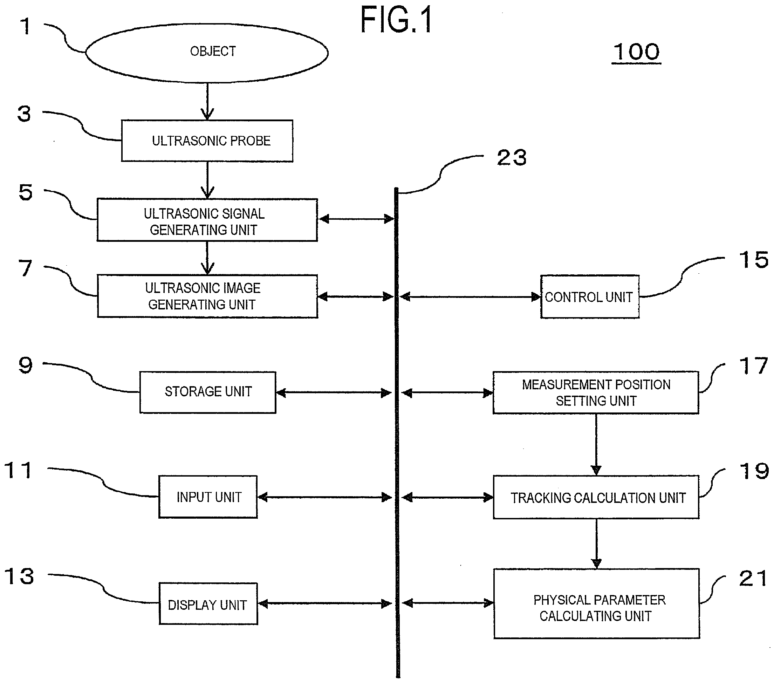

Fig. 1 is a block diagram showing the general configuration of the ultrasonic diagnostic apparatus in the present embodiment. As shown in Fig. 1, an ultrasonic diagnostic apparatus 100 of the present embodiment comprises an ultrasonic probe 3 configured to transmit/receive ultrasonic waves to/from an object 1, an ultrasonic signal generating unit 5 configured to generate ultrasonic signals on the basis of the reflected echo signals measured by the ultrasonic probe 3, an ultrasonic image generating unit 7 configured to generate an ultrasonic image on the basis of ultrasonic signals, a storage unit 9 configured to store the generated ultrasonic image or various programs for controlling the ultrasonic diagnostic apparatus, an input unit 11 to be the input interface, a display unit 13 to be the output interface, a control unit 15 configured to control the respective units of the ultrasonic diagnostic apparatus, a measurement position setting unit 17 configured to set a region of interest in biological tissue of a diagnostic target region on the ultrasonic image which is displayed on the display unit 13, a tracking calculation unit 19 configured to track the movement of biological tissue in a diagnostic target region in a plurality of measurement points of a region of interest, a physical quantity calculating unit 21 configured to calculate a specific physical quantity on the basis of the tracking result, and a system bus 23 configured to connect the respective units.

The ultrasonic probe 3 transmits/receive ultrasonic waves to/from the object 1, provided with a scanning method such as a linear type, convex type or sector type. These types of ultrasonic probe can be provided with configuration and function in which transducers are arrayed one-dimensionally to acquire 2-dimensional signals, transducers are arrayed 2-dimesionally to acquire 3-dimensional signals, or transducers are arrayed one-dimensionally to acquire 3-dimensional signals by mechanically executing special scanning.

The ultrasonic signal generating unit 5 transmits and receives ultrasonic waves that are converted into electric signals between the unit and the ultrasonic probe 3. It receives the information on the power or timing of transmission and reception from the control unit 15, so that the transmission and reception are controlled to acquire desired ultrasonic waves. The ultrasonic signal generating unit 5 also executes signal processing via a phasing and adding unit or an amplifier circuit on the signals received from the ultrasonic signal transmission/reception unit in conformity to the imaging setting, and acquires the shaped ultrasonic signals. These signals are stored in the storage unit 9 to be used for the subsequent measurement.

The ultrasonic image generating unit 7 generates an image of biological tissue of the object 1. The ultrasonic signals passed through the ultrasonic signal generating unit are input, and an ultrasonic image based on the imaging setting of the apparatus is generated. These signals are stored in the storage unit 9 for using the subsequent measurement.

The storage unit 9 stores the programs for operating various systems which configure the ultrasonic diagnostic apparatus 100 and data such as signal data, image data and measurement data, and reading and writing are executed in response to the processing.

The input unit 11 is the interface for executing various operations of the diagnostic apparatus. It is an input device such as a keyboard, trackball, switch or dial, and is used for performing operation to obtain images, specifying a region of interest of biological tissue, or executing various measurement settings.

The display unit 13 displays a region of interest, measurement values, and an ultrasonic image on a screen, or outputs the measurement values as a measurement report. The control unit 15 controls the entire system. For example, a device such as a CPU is used as the control unit.

The measurement position setting unit 17 sets a region of interest on an ultrasonic image. For example, it segments the region of interest into a meshed pattern, and executes the process to set the intersecting points of segmenting lines as the measurement points. The measurement position setting unit 17 will be described later in detail. On the ultrasonic screen, the ultrasonic image which is read out from the storage unit 9 is displayed. Since the images are stored in time-series order, the frame of a desired time phase can be selected and displayed using an input device. If an image is obtained by synchronizing with biological signals (for example, an electrocardiogram), it may also be configured, for example so that an image of the R-wave phase of an electrocardiogram can be automatically selected.

The tracking calculation unit 19 performs calculation for tracking the movement of biological tissue using the ultrasonic signal near the position of the measurement point or the amplitude information of the image, and calculates the displacement thereof. The information on the ultrasonic signal and the image which is stored in the storage unit 9 is read in and used for the calculation. The starting frame and the ending frame of the tracking calculation may be set by a user using an input device, or if an image is obtained by synchronizing with a biological signal (for example, an electrocardiogram), the frame group may be automatically limited, for example in the range from an R-wave to the next R-wave of an electrocardiogram.

The physical calculation unit 21 calculates the physical quantity such as velocity, strain, area and volume as time-series information on the basis of the displacement, in accordance with the measurement items appointed by an examiner using the input device. The calculated physical quantity is displayed by the display unit 13 on an ultrasonic image by pseudocolor display or numeric values, or output as a measurement report file. The processing flow and characteristic configuration of the ultrasonic diagnostic apparatus in the present embodiment will be described in detail for each embodiment.

### Embodiment 1

The first embodiment is an example, by citing a cardiac short axis view, of spatial, temporal and consecutive measurement of the inside of cardiac muscle and calculation of a measurement value thereof for each local portion. Here, the measurement target will be described as a 2-dimensional ultrasonic signal and a 2-dimensional ultrasonic image.

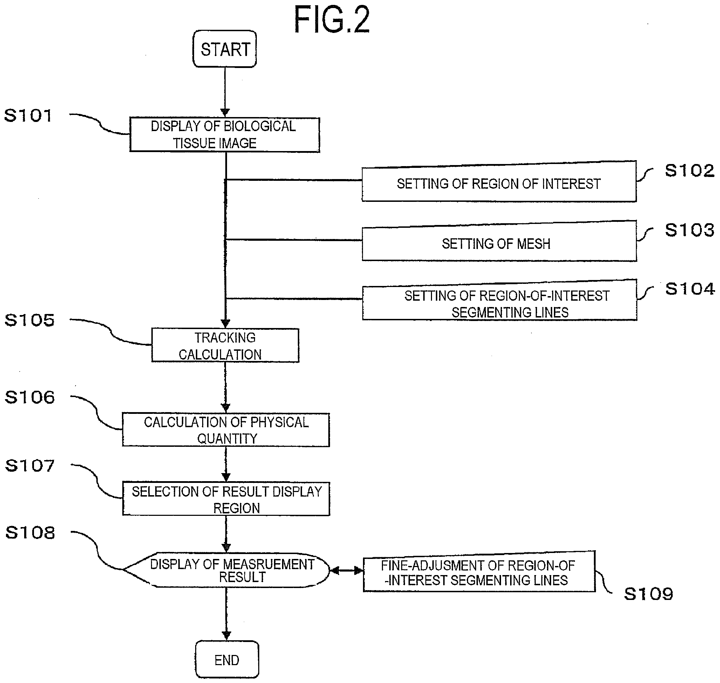

Fig. 2 is a flowchart showing the processing flow of the ultrasonic diagnostic apparatus in the present embodiment. As shown in Fig. 2, the examiner first images and displays an ultrasonic image of biological tissue (cardiac muscle) (S101). The ultrasonic transmitting/receiving surface of the ultrasonic probe 3 is applied to an object 1, and a region including the measurement target is imaged. In order to execute tracking, images are obtained for a certain period of time. If biological signals (for example, electrocardiogram) are input, the image may also be obtained by synchronizing with the input signals. For example, one heartbeat from an R-wave to the next R-wave of the electrocardiogram may be automatically obtained, or the starting frame and the ending frame may be specified with respect to the frame group in a certain interval after the image is obtained. While the specified frame group is stored in the storage unit 9, when the tracking calculation is to be executed using ultrasonic signals, the ultrasonic signals are also stored in the storage unit 9 in the same manner as the ultrasonic images to be read out for the calculation.

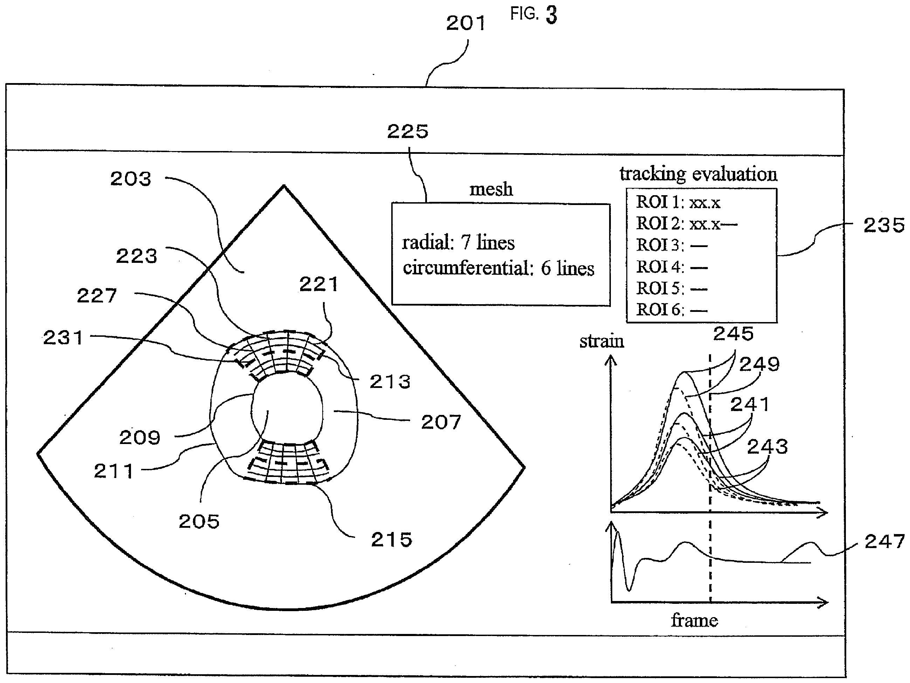

Fig. 3 is an example of a display screen of the ultrasonic diagnostic apparatus. On a display screen 201 of the ultrasonic diagnostic apparatus, an ultrasonic image 203 is displayed on the left side of the screen, and the cardiac short axis view is displayed here. More specifically, on the ultrasonic image 203, a short axis view is displayed including a heart chamber 205 of a heart of an object and a cardiac muscle 207 of a doughnut shape surrounding the heart chamber 205. Further, an endocardium 209 exists on the border between the cardiac muscle 207 and the heart chamber 205 along the circumferential direction, and an epicardium 211 exists on the border between the cardiac muscle 207 and the other tissue on the outer circumferential side of the cardiac muscle 207 along the circumferential direction.

Next, the examiner sets a region of interest by the measurement position setting unit 17 (S102). The setting is executed using an input device on the ultrasonic image displayed in S101. Fig. 3 shows an example of a screen for setting a region of interest. A short axis view is depicted on the screen, and zonal regions of interest 213 and 215 are set on the image thereof. As for the setting method, an existing automatic setting method based on the region segmentation method may be used, or the contour of cardiac muscle may be manually traced. While zonations to surround a cardiac muscle are used here, the setting method of the present invention is not limited thereto. The examiner may also set a region of interest in a desired target portion itself, for example a tumor or a part of the inside of cardiac muscle, without surrounding the region by the border of cardiac muscle. Also, the number of regions of interest may be set as one or plural numbers. For example, in the doughnut-shaped cardiac muscle in the cardiac short axis view, a plurality of partial regions in cardiac muscle can be separated and set as regions of interest. In this manner, a specific physical quantity of cardiac muscle can be compared and observed in plural regions of interest. In the example of Fig. 3, a first region of interest 213 which is close to the transmission/reception surface of the ultrasonic probe 3 and a second region of interest 215 which is on the far side are set as separate regions of interest, with the heart chamber 205 interposed therebetween.

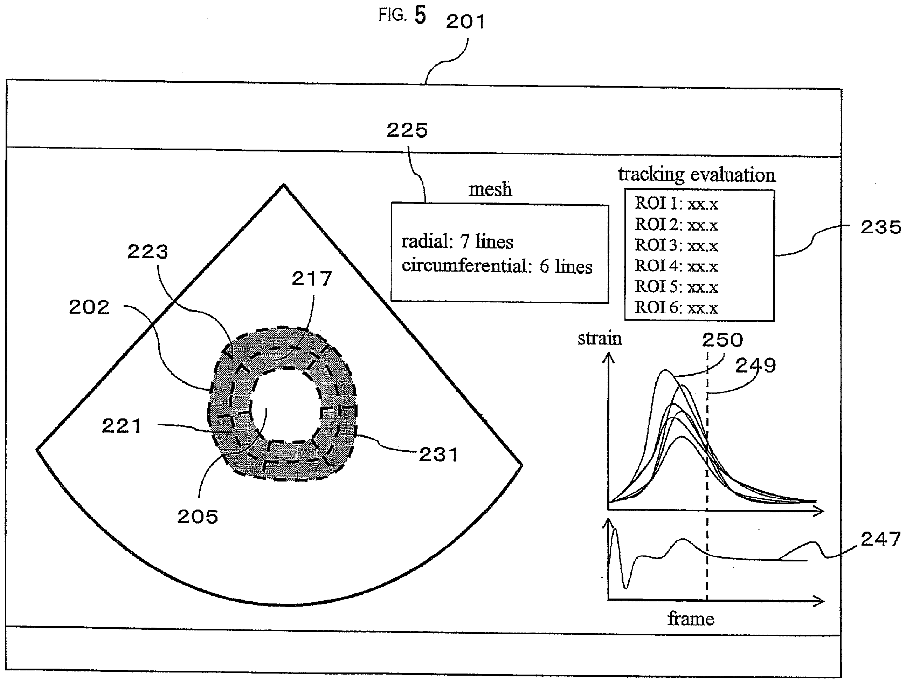

Fig. 5 is another example of a display screen of the ultrasonic diagnostic apparatus. As shown in Fig. 5, by setting a doughnut-shaped region of interest 217 which surrounds the doughnut-shaped entire cardiac muscle, and the set region of interest may be divided into six segments based on the 17-segment method which is recommended by ASE (American Society of Echocardiography).

Next, the examiner sets the method of mesh division by the measurement position setting unit 17 (S103). The mesh division method can be adapted to different measurement items of target tissue. Since cardiac muscle is the target in this case, as shown in Fig. 3, the directions for segmenting the regions into meshed pattern are set in the circumferential direction and the radial direction. That is, the objective of segmentation is to measure the expansion and contraction in the circumferential direction and the radial direction of the cardiac muscle. In other words, the regions of interest are segmented into meshed pattern by a plurality of first segmenting lines 221 along the epicardium 211 and the endocardium 209 of the cardiac muscle and a plurality of second segmenting lines 223 that are orthogonal to the plurality of first segmenting lines 221, and a plurality of intersecting points of the first segmenting lines 221 and the second segmenting lines 223 are set as measurement points respectively. The density of the mesh, i.e. the number of the first segmenting lines 221 and the second segmenting lines 223 with respect to a certain region can be arbitrarily adjusted.

As the number of mesh segmentation increases, measurement can be performed in more detail and smoother luminance variation can be depicted in pseudocolor display. The measurement may also be executed on the basis of the distance between the inner membrane side and the outer membrane side as the conventional method without performing any segmentation. The setting of the number of segmentation may be freely selected by the examiner using an input device. The set values are displayed on a mesh setting display 225.

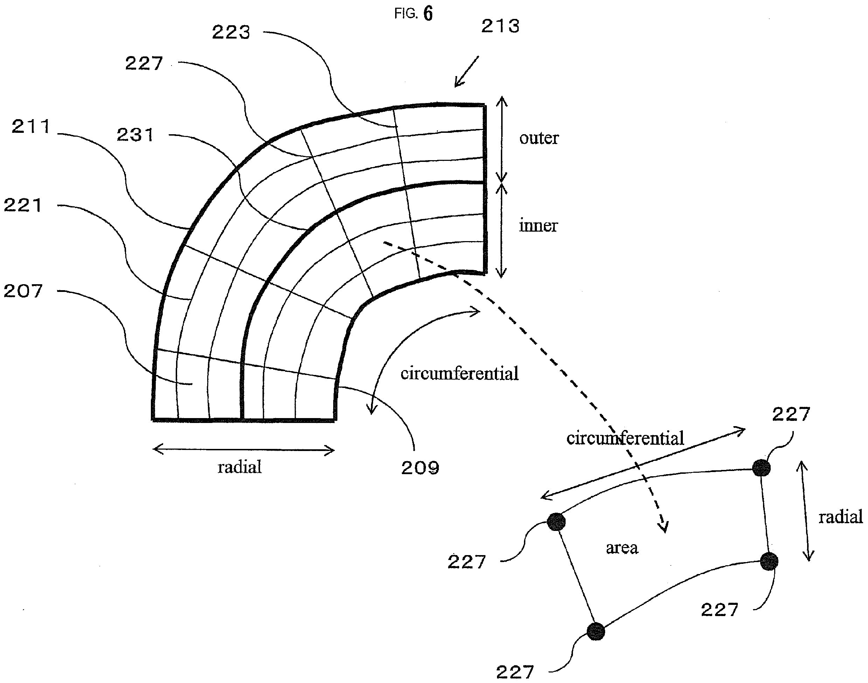

Here, seven first segmenting lines 221 are used for segmenting the cardiac muscle in the radial direction, and six second segmenting lines 223 are used for segmenting the cardiac muscle in the circumferential direction. These segmenting lines cross each other, and the intersecting points are set as measurement points 227. Fig. 6 is an enlarged view of a region of interest. The lower right part of Fig. 6 is a view showing a segment which is abstracted from a region of interest. The measurement points 227 are set on the contact points of the segments, i.e. on the intersecting points of the meshed pattern. Therefore, 7×6=42 of measurement points are set in this case.

Next, the examiner sets a region-of-interest segmenting lines 231 by the measurement position setting unit 17 (S104). The region-of-interest segmenting lines 231 are to be set when the inside of a region of interest is desired to be further segmented into groups (a region of interest is divided into two groups in this example). While the region-of-interest segmenting line 231 is set at approximately intermediate position between the endocardium and the epicardium since it is expected that the property of cardiac muscle is different in the endocardium and the epicardium as shown in Figs. 3 ∼ 6, it should be configured as freely settable in conformity to the target of measurement. For example, the region-of- interest segmenting line 231 can be set to surround a tumor.

Also, while the case that one of the plurality of first segmenting lines 221 that are set in a region of interest is set as the region-of-interest segmenting line 231 is exemplified in the present embodiment, the present invention is not limited thereto. For example, one of the plurality of second segmenting lines 223 can be set as a region-of-interest segmenting line. Also, a line other than the first segmenting lines 221 or the second segmenting lines 223 can also be set as a region-of-interest segmenting line.

Next, the ultrasonic diagnostic apparatus measures the movement of a measurement-point group by the tracking calculation unit 19 (S105). The tracking calculation unit tracks a plurality of measurement points using the first segmenting lines 221 and the second segmenting lines 223. That is, the tracking calculation unit tracks the plurality of measurement points that are set on the basis of the first segmenting lines 221 and the second segmenting lines 223. For example, the intersecting points of the first segmenting lines 221 and the second segmenting lines 223 may be respectively set as measurement points as described above, or a predetermined position within the respective lattices formed by the first segmenting lines 221 and the second segmenting lines 223 may also be set as measurement points. As for the tracking method, a commonly-known method can be used such as the correlation method or optical flow method. The target signal is the ultrasonic signal or ultrasonic image stored in the storage unit 9.

Since the measurement points are set in high density, the method for improving the resolution may also be used.

For example, the tracking calculation can be adopted by presumptively improving the resolution of a target image. Also, the tracking calculation can be adopted while recursively changing the block size for a block matching. The method of adopting the tracking calculation in which the correlation method and the optical flow method are combined can also be used. The time phase for the measurement is the frame group of an ultrasonic image acquired and set in S101. As the tracking result, the data of positional coordinates in the respective measurement points of each frame is obtained.

Next, the ultrasonic diagnostic apparatus executes calculation of physical quantity by the physical quantity calculating unit 21 (S106). As shown in the lower right part of Fig. 6, with respect to the group of measurement points 227, the physical quantity is calculated based on the distance between two adjacent points. For example, the distance between the two points and the rate of change (strain) from the initial distance are calculated. The radial strain is calculated in the radial direction, and the circumferential strain is calculated in the circumferential direction. Also, the area strain may be calculated on the basis of the change of area which is framed in by the four points. In this manner, the physical quantity of the respective small regions in the meshed pattern is calculated. Further, the average value of these physical quantities in the entire meshed pattern is calculated as the measurement value of the region of interest. Also, the average value is calculated in the epicardium and the endocardium that are divided by the region-of-interest segmenting line 231, and set as the measurement values respectively.

Next, the examiner or the apparatus selects a result display region (S107). The selection criterion is the accuracy of tracking. Here, the evaluation value is calculated for self-evaluation of the tracking accuracy. Generally, the tracking accuracy tends to be lowered as the resolution is increased. Especially ultrasonic images have tendency to be under adverse condition with respect to the tracking calculation due to existence of artifacts or low resolution. Therefore, the tracking cannot always be calculated accurately depending on the condition. As for the evaluation value, the method based on the error-vector detection is used.

For example, since there are many measurement points, the quantity of error-vectors is digitalized using the method of determining that the displacement of a vector at a certain measurement point is different (in error) compared to the surrounding displacement vectors.

Since the evaluation value in the respective measurement points can be obtained, the values may also be calculated either in the entire region of interest or by dividing the region of interest using the region-of-interest segmenting line into the endocardium side and the epicardium side. The examiner selects a result display area while checking the magnitude of evaluation value so that the region of interest with low measurement accuracy will not be displayed. Also, the apparatus may automatically make a selection on the basis of the previously set threshold value.

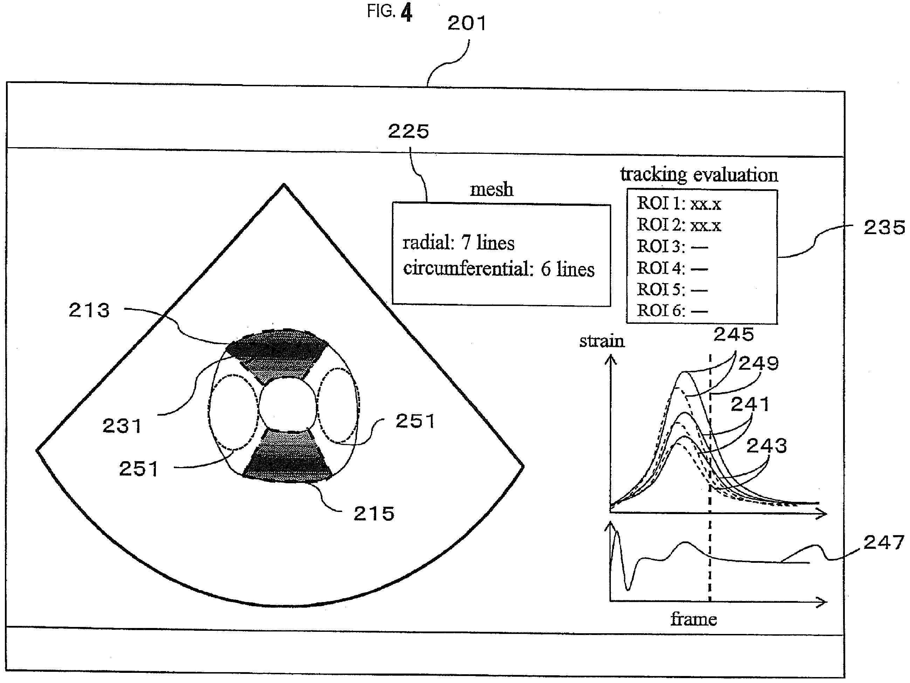

Next, the apparatus displays the measurement result by the display unit 13 (S108). Fig. 4 is an example of a display screen showing the measurement result. The physical quantity is converted into the luminance value for pseudocolor display. These luminance values are superimposed over the meshed pattern of the region of interest and interpolated for smooth luminance variation, so as to color the entire region of interest. In Fig. 4, the coloring is performed in all of the regions of interest 213 and 215. The self-evaluation value of the tracking is displayed on the chart of a tracking self-evaluation result 235.

The examiner checks the magnitude of these numeric values and determines whether to adopt the measurement result. A graph is displayed on the right side of Fig. 4. The graph is displayed for each local region in the region of interest, for example as a strain value 241 of the entire region of interest, a strain value 243 of the endocardiam-side region and a strain value 245 of the epicardium-side region. A biosignal 247 is also displayed. A time phase bar 249 is further provided to the time phase of the currently displayed image for movie display. In the case that six regions of interest are set as shown in Fig. 5, each region of interest is pseudocolor-displayed and the number of graphs 250 to be displayed will be the same as the number of regions of interest. Here, the graphs of the first region of interest 213 are denoted by solid lines, and the graphs of the second region of interest 215 are denoted by dotted lines. It may also be operated, when the tracking self-evaluation result 235 is observed on a certain region of interest and determined that the measurement result thereof is not to be adopted, not to color-display only that region of interest.

Next, the examiner fine-adjusts the region-of-interest segmenting line 231 by the measurement position setting unit 17 (S109). The examiner adjusts the position of the region-of-interest segmenting line while observing the result of movement tracking. The measurement result in the local regions changes when the region-of-interest segmenting line is adjusted, thus the adjustment is made so that this change is reflected on the graph and the measurement result of a desired position can be obtained while observing the graph.

As described above, in accordance with the present embodiment, it is possible to set a region of interest in conformity with the property of biological tissue, and the movement of the inside of biological tissue can be tracked by setting measurement points in a meshed pattern. By segmenting a region of interest by the region-of-interest segmenting line 231 and setting the segmented regions as local regions or pseudocoloring the measurement values, the difference of the local property in the inside of biological tissue can be easily discriminated. Also, measurement accuracy can be improved by segmenting the region of interest into plural regions such as a first region and a second region so as to set them at measurement positions in conformity to the effected area of an object or set them at the places where sufficient image quality can be provided. Also, by segmenting the region of interest and setting only necessary portions for measurement, calculation amount can be reduced compared to tracking the entire cardiac muscle, thereby improving the examination efficiency.

More specifically, in the cardiac short axis view, when a region of interest is set on the cardiac regions of a doughnut-shaped cardiac muscle other than the region which is close to the transmitting/receiving surface of the ultrasonic probe and the region which is away from the surface, with the heart chamber interposed therebetween (for example, cardiac muscle regions 251 in Fig. 4), the image quality of an ultrasonic image will not be sufficient due to influence of noise, etc., which could lead to the lowering of accuracy in the movement tracking. Therefore, even when the physical quantity is calculated in these cardiac muscle regions, the calculated quantity may lack credibility. If the region of interest is set as a doughnut-shaped pattern despite the above-described tendency, the measurement will be executed even in the regions which lack credibility, thus is not preferable in view of calculation efficiency. In view of this, by segmenting the doughnut-shaped cardiac muscle in a cardiac short axis view and setting a region which is close to the transmitting/receiving surface of an ultrasonic probe and a region which is away from the surface with the heart chamber interposed therebetween as shown in Fig. 3, it is possible to improve the efficiency in calculation process and to enable contrast observation between the region which is close to the transmitting/receiving surface of the ultrasonic probe and the region which is away from the surface, with the heart chamber interposed therebetween.

Also, measurement points are appropriately set in the present embodiment, in conformity to the property or expansion and contraction of the cardiac muscle. That is, multiple measurement points are set along the main expanding and contracting directions (the circumferential direction and the irradiating direction (radial direction)) of the cardiac muscle. In accordance with the present embodiment, since measurement points are set along the expanding and contraction direction of the cardiac muscle as described above, in the case, for example that the variation of distance is measured between the adjacent measurement points on a first segmenting line 221 or between the adjacent measurement points on a second segmenting line 223, the measurement values are useful for making an accurate diagnosis since the property of the respective regions in the cardiac muscle is appropriately reflected to the measurement values. Also, the all of the distances from the endocardium 209 and the epicardium 211 become constant in relation to the respectively set plural measurement points on the plurality of first segmenting lines 221. Therefore, for example, in the case that the variation of distance is measured from the respective measurement points on a certain first segmenting line 221 to the endocardium 209 or the epicardium 211, the property of the respective regions in the cardiac muscle can be appropriately compared by comparing the respective measurement values, thus the measurement results are useful for making an accurate diagnosis. In this manner, in accordance with the present embodiment, it is possible to measure expansion and contraction of cardiac muscle to be reflected to the measurement values of physical quantity, which enables accurate comparison of measurement values for making a proper diagnosis.

### Embodiment 2

The second embodiment is the method, citing an example of a cardiac short axis view, which spatially, temporally and consecutively measures the inside of cardiac muscle and calculates the measurement value for each local region of the cardiac muscle. The present embodiment is different from the first embodiment in that the measurement target is a 3-dimensional signal and a 3-dimensional image. Therefore, the difference from the first embodiment will be mainly described in this embodiment, by omitting the duplicative description as the first embodiment such as the apparatus configuration and the processing procedure.

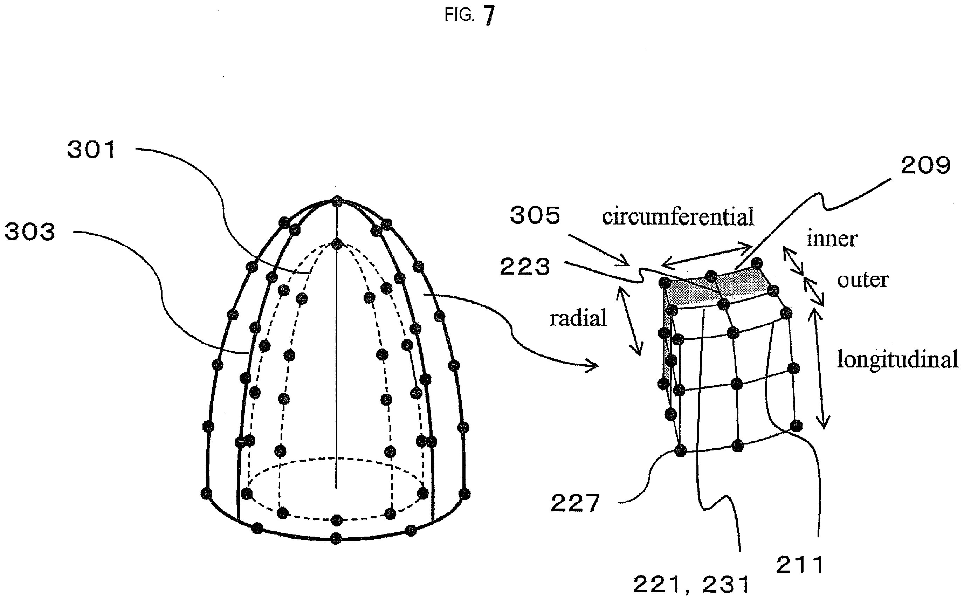

First, a 3-dimensional ultrasonic image is displayed on the basis of the ultrasonic signals of the biological tissue in plural cross sections of an object (S101), and the setting of a region of interest is carried out (S102). Here, the region of interest may be manually set on a 3-dimensional ultrasonic image using an input device, or may automatically set using a conventional automatic region segmentation method. In this manner, the setting of a 3-dimensional region of interest is executed. Fig. 7 is a view showing an example of the measurement method in a 3-dimensional ultrasonic image, and the left part of Fig. 7 shows the extracted view of an endocardium contour 301 and an epicardium contour 303 of the entire left ventricle. The right part of Fig. 7 is the extracted view of a certain 3-dimensional region of interest 305 which is set on a 3-dimensional ultrasonic image. As shown in the diagram, the cardiac muscle has, for example a 10mm thick campanulate shape.

In the setting of a meshed pattern (S103), the segmentation is performed in the three directions of the radial direction, short-axis direction and the long-axis direction. That is, the objective of this segmentation method is to perform measurement in the respective radial, circumferential and longitudinal directions. In other words, in a certain cross section which is orthogonal to the longitudinal direction, the region of interest is segmented into a meshed pattern by a plurality of first segmenting lines 221 along the epicardiam 211 and the endocardium 209 of the cardiac muscle and a plurality of second segmenting lines 223 that are orthogonal to the plurality of first segmenting lines 221. Further, by elongating the first segmenting lines 221 and the second segmenting lines 223 along the epicardiam and the endocardium in the longitudinal direction and cutting the cross section that are orthogonal to the longitudinal direction at predetermined intervals in the direction thereof, the region of interest is segmented into a meshed pattern. The intersecting points of these segmenting lines are set as measurement points 227.

Also, by setting of a region-of-interest segmenting line 231 (S104), the 3-dimensional region of interest is segmented into local regions. For example, as shown in the right part of Fig. 7, the region-of-interest segmenting line 231 is set for dividing the region of interest into the endocardium side and the epicardium side. Here, after the region-of-interest segmenting line 231 is set, the 3-dimensional region of interest is segmented into a 3-dimensional region on the endocardiac side and a 3-dimensional region on the epicardiac side by the plane of which the region-of-interest segmenting line 231 is elongated along the endocardiam and the epicardium in the longitudinal direction.

Next, the apparatus executes the measurement of movement with respect to the respective measurement points 227 in the 3-dimensional signal and the 3-dimensional ultrasonic image (S105), and calculates the physical quantity (S106). The physical quantity is the distance in the respective radial, circumferential and longitudinal directions, the strain based on the distance variation, the area of a certain cross section in a 3-dimensional region of interest, the volume of a 3-dimensional region of interest, and so on. The examiner or the apparatus selects a result display region on the basis of the self-evaluation of movement tracking (S107). In the display of measurement result (S108), the measurement values are converted into luminance values, and the surface of the region of interest is colored. Then the examiner adjusts the position of the region-of-interest segmenting line while viewing the graph which indicates the result of movement tracking (S109).

As described above, in accordance with the present embodiment, since measurement values are appropriately set in conformity to the 3-dimensional property or extraction and contraction of cardiac muscle, it is possible to measure expansion and contraction of cardiac muscle which facilitates accurate reflection of expansion and contraction of cardiac muscle to the respective measurement values for making a proper diagnosis, as in the first embodiment. Also, the setting of a region of interest can be coincided with the property of biological tissue in a 3-dimensional space, which enables the tracking of movement inside of the biological tissue. Addition of the depth dimension in measurement also enables simultaneous measurement in three directions, which improves measurement accuracy compared to the 2-dimensional analysis.

Moreover, by separating a region of interest by a region-of-interest segmenting line and setting it as a local region or pseudocoloring the measurement values, the difference of local properties in the biological tissue can be easily discriminated.

The ultrasonic diagnostic apparatus of the present embodiment comprises in its basic configuration:
an ultrasonic probe configured to transmit/receive ultrasonic waves to/from an object;
an ultrasonic signal generating unit configured to generate an ultrasonic signal on the basis of the reflected echo signal, which is received by the ultrasonic probe, in the cross section of the tissue including the cardiac muscle of the heart of the object;
an ultrasonic image constructing unit configured to construct an ultrasonic image on the basis of ultrasonic signals;
a display unit configured to display the ultrasonic image;
a measurement position setting unit configured to set a region of interest in the cardiac muscle on the ultrasonic image displayed on the display unit;
a tracking calculation unit configured to track the movement of cardiac muscle in a plurality of measurement points in a region of interest; and
a physical quantity calculating unit configured to calculate a specific physical quantity on the basis of the tracking result,
and displays the calculated physical quantity on the display unit. As for a specific physical quantity, at least one of the myocardial velocity in plural measurement points of a region of interest, the strain of the cardiac muscle in a plurality of measurement points in a region of interest, the area of cardiac muscle which is framed by a region of interest and, in the case that a 3-dimensional ultrasonic image is generated, the volume of cardiac muscle which is surrounded by a 3-dimensional region of interest can be cited.

In order to solve the previously described problem, the tracking calculation unit is characterized in tracking the plurality of measurement points by a plurality of first segmenting line along the epicardium and the endocardium of the cardiac muscle and the second segmenting lines that are orthogonal to the first segmenting lines. For example, it may be configured so that plural measurement points are set on the basis of plural first segmenting lines and plural second segmenting lines, so that the set plural measurement points are tracked for measurement. More specifically, the region of interest is segmented into a meshed pattern by plural first segmenting lines and plural second segmenting lines, for setting the intersecting points of the first segmenting lines and the second segmenting lines as plural measurement points.

In order to improve accuracy in diagnosis of a specific physical quantity of cardiac muscle, proper setting of measurement points in conformity to the property or the expanding/contracting direction of the cardiac muscle is necessary. In this regard, the present invention sets plural measurement points along the main expanding and contracting direction of the cardiac muscle (for example, if an ultrasonic image shows the cardiac short axis view of an object including the heart chamber and the cardiac muscle surrounding the heart chamber forming a doughnut shape, the circumferential direction and the irradiating direction (radial direction) of the cardiac muscle). In this manner, since measurement points are set along the expanding and contracting direction of the cardiac muscle, in the case, that the distance variation between the adjacent measurement points on a first segmenting line or the adjacent measurement points on a second segmenting line and the like is measured, the property of the respective regions of the cardiac muscle is adequately reflected to the respective measurement points, which makes the measurement values useful for making an accurate diagnosis. Also, all of the distances from the endocardium or the epicardium to the plurality of measurement points that are respectively set on a plurality of first segmenting lines become constant. Therefore, in the case, for example that the distance variation from the respective measurement points on a certain first segmenting line to the endocardium or the epicardium is measured, the property of the respective regions in the cardiac muscle can be appropriately compared by comparing the measurement values, which is useful for making an accurate diagnosis.

Also, in the case an ultrasonic image to be displayed on the display unit is a cardiac short axis view, the measurement position setting unit can set a region of interest in a doughnut shape in conformity to the property of the doughnut-shaped cardiac muscle in the short axis view of the heart. On the other hand, the measurement position setting unit can set a plurality of regions of interest by separating them to a plurality of partial regions in the doughnut-shaped cardiac muscle of the cardiac short axis view. It is preferable to set plural regions of interest in this manner for comparing and studying specific physical quantities in the respective regions of interest. In this manner, this method of dividing a region of interest into plural separated regions is preferable for performing contrast observation of the specific physical quantities in the respective regions of interest. The measurement position setting unit is also capable of segmenting the doughnut-shaped cardiac muscle in the short axis view of the heart into the cardiac muscle on the side which is close to the transmitting/receiving surface of an ultrasonic probe and the cardiac muscle on the far side with the heart chamber interposed therebetween as separate regions of interests.

In the cardiac short axis view, the image quality of an ultrasonic image is not sufficient due to influence of noise, etc. in cardiac regions of a doughnut-shaped cardiac muscle other than the region which is close to the transmitting/receiving surface of the ultrasonic probe and the region which is away from the surface with a heart chamber interposed therebetween, which can lower the accuracy of the movement tracking. Therefore, even when the physical quantity is calculated in these cardiac muscle regions, the calculated values may lack credibility. If the region of interest is set as a doughnut-shaped pattern despite of the above-described tendency, the measurement will be executed even in the regions which lack credibility, which is not preferable in view of calculation efficiency. In view of this, by separating the doughnut-shaped cardiac muscle in a cardiac short axis view and setting a region which is close to the transmitting/receiving surface of an ultrasonic probe and a region which is away from the surface with the heart chamber interposed therebetween, it is possible to improve the efficiency in calculation process and to enable contrast observation of the region which is close to the transmitting/receiving surface of the ultrasonic probe and the region which is away from the surface with the heart chamber interposed therebetween.

Also, the measurement position setting unit is capable of setting one of the plurality of first segmenting lines or the plurality of second segmenting lines that are set in a region of interest as a region-of-interest segmenting line for dividing the region of interest into two regions, or setting a region-of-interest segmenting line, on an ultrasonic image, which is separate from the plurality of first segmenting lines or the plurality of second segmenting lines. In this manner, it is possible to easily divide a region of interest which is once set, which facilitates the convenience in evaluating the measurement values of endocardiac side and the epicardiac side of the cardiac muscle separately or evaluating the normal region and the abnormal region separately.

### Description of Reference Numerals

- 1:: object
- 3:: ultrasonic probe
- 5:: ultrasonic signal generating unit
- 7:: ultrasonic image generating unit
- 11:: input unit
- 13:: display unit
- 15:: control unit
- 17:: measurement position setting unit
- 19:: tracking calculation unit
- 21:: physical quantity calculating unit
- 100:: ultrasonic diagnostic apparatus
- 203:: ultrasonic image
- 205:: heart chamber
- 207:: cardiac muscle
- 209:: endocardium
- 211:: epicardium
- 213:: first region of interest
- 215:: second region of interest
- 221:: first segmenting line
- 223:: second segmenting line
- 227:: measurement point
- 231:: region-of-interest segmenting line
- 305:: 3-dimensional region of interest

## Claims

1. An ultrasonic diagnostic apparatus (100) comprising:
an ultrasonic probe (3) configured to transmit/receive ultrasonic waves to/from an object to be examined (1);
an ultrasonic signal generating unit (5) configured to generate an ultrasonic signal on the basis of the reflected echo signal, which is received by the ultrasonic probe (3), in the cross section of the tissue including the cardiac muscle (207) of the heart of the object (1);
an ultrasonic image generating unit (7) configured to generate an ultrasonic image on the basis of the ultrasonic signal;
a display unit (13) configured to display the ultrasonic image;
a measurement position setting unit (17) configured to set a region of interest (213, 215, 217, 305) in the cardiac muscle (207) on the ultrasonic image displayed on the display unit (13);
a tracking calculation unit (19) configured to track the movement of cardiac muscle (207) in a plurality of meshed pattern measurement points (227) in the region of interest (213, 215, 217, 305); and
a physical quantity calculating unit (21) configured to calculate a specific physical quantity on the basis of the tracking result,
wherein:
the calculated physical quantity is displayed on the display unit (13); and
the tracking calculation unit (19) tracks the plurality of meshed pattern measurement points (227) using
a plurality of first segmenting lines (221) along the epicardium (211) and the endocardium (209) of the cardiac muscle (207) and a plurality of second segmenting lines (223) that are orthogonal to the plurality of first segmenting lines (221),
**characterised in that**
the plurality of measurement points (227) are set on every intersections of the first segmenting lines (221) and the second segmenting lines (223), and
wherein the physical quantity calculating unit (21) calculates both a radial strain and a circumference strain by using every two adjacent measurement points (227).

2. The ultrasonic diagnostic apparatus (100) according to claim 1, wherein:
the measurement position setting unit (17) is configured to segment the region of interest (213, 215, 217, 305) by the plurality of first segmenting lines (221) along the epicardium (211) and the endocardium (209) of the cardiac muscle (207) and the plurality of second segmenting lines (223) that are orthogonal to the plurality of first segmenting lines (221); and
to set the intersecting points of the first segmenting lines (221) and the second segmenting lines (223) as the plurality of measurement points (227).

3. The ultrasonic diagnostic apparatus (100) according to claim 1, wherein the measurement position setting unit (17), in the case that the ultrasonic image to be displayed on the display unit (13) is a cardiac short axis view of the object (1) including the heart chamber (205) and the cardiac muscle (207) surrounding the heart chamber (205), is configured capable of setting a region of interest (217) by segmenting the cardiac muscle (207) in the cardiac short axis view into plural partial regions.

4. The ultrasonic diagnostic apparatus (100) according to claim 1, wherein the measurement position setting unit (17), in the case that the ultrasonic image to be displayed on the display unit (13) is a cardiac short axis view of the object (1) including the heart chamber (205) and the cardiac muscle (207) surrounding the heart chamber (205), is configured capable of setting the region of interest (213, 215) by separating the cardiac muscle (207) in the cardiac short axis view into the cardiac muscle (207) which is on the side close to the transmitting/receiving surface of the ultrasonic probe (3) and the cardiac muscle (207) which is on the side away from the transmitting/receiving surface, with the heart chamber (205) interposed therebetween.

5. The ultrasonic diagnostic apparatus (100) according to claim 1, wherein the measurement position setting unit (17) is configured capable of setting one of the plurality of first segmenting lines (221) or one of the plurality of second segmenting lines (223) that are set in the region of interest (213, 215, 217, 305) as a region-of-interest segmenting line (231) for dividing the region of interest (213, 215, 217, 305) into two regions, or setting the region-of-interest segmenting line (231) on an ultrasonic image separately from the plurality of first segmenting lines (221) or the plurality of second segmenting lines (223).

6. The ultrasonic diagnostic apparatus (100) according to claim 1, wherein:
the ultrasonic probe (3) has configuration in which a plurality of transducers that transmit/receive ultrasonic waves to/from the object (1) are two-dimensionally arrayed or the plurality of transducers are one-dimensionally arrayed for mechanically executing spatial scanning so as to measure the reflected echo signals in plural cross sections of the tissue including the cardiac muscle (207) of the heart of the object (1);
the display unit (13) is configured to display a 3-dimensional ultrasonic image generated on the basis of the ultrasonic signals in the plural cross sections; and
the measurement position setting unit (17) sets a 3-dimensional region of interest (305) on the 3-dimensional ultrasonic image displayed on the display unit (13).

7. The ultrasonic diagnostic apparatus (100) according to claim 1, wherein the specific physical quantity is at least one of the myocardial velocity in a plurality of measurement points (227) in the region of interest (213, 215, 217, 305), the strain of the cardiac muscle (207) in the plurality of measurement points (227) in the region of interest (213, 215, 217, 305), the area of the cardiac muscle (207) which is framed by the region of interest (213, 215, 217), and the volume of the cardiac muscle (207) which is framed by a 3-dimensional region of interest (305).

8. A measurement point tracking method using an ultrasonic diagnostic apparatus (100), said method comprising:
transmitting ultrasonic waves to an object to be examined (1) by an ultrasonic probe (3);
receiving ultrasonic waves reflected from said object (1);
generating an ultrasonic signal by an ultrasonic signal generating unit (5) on the basis of the reflected echo signal, which is received by the ultrasonic probe (3), in the cross section of the tissue including the cardiac muscle (207) of the heart of the object (1);
generating an ultrasonic image by an ultrasonic image generating unit (7) on the basis of the ultrasonic signal;
displaying the ultrasonic image by a display unit (13);
setting a region of interest (213, 215, 217, 305) by a measurement position setting unit (17) in the cardiac muscle (207) on the ultrasonic image which is displayed on the display unit (13);
tracking the movement of cardiac muscle (207) in a plurality of meshed pattern measurement points (227) in the region of interest (213, 215, 217, 305) by a tracking calculation unit (19);
calculating a specific physical quantity by a physical quantity calculating unit (21) on the basis of the tracking result; and
displaying displays the calculated physical quantity on the display unit (13), wherein the tracking step comprises tracking the plurality of meshed pattern measurement points (227) by the tracking calculation unit (19) using a plurality of first segmenting lines (221) along the epicardium (211) and the endocardium (209) of the cardiac muscle (207) and a plurality of second segmenting lines (223) that are orthogonal to the plurality of first segmenting lines (221),
**characterized in that**
the plurality of measurement points (227) are set on every intersections of the first segmenting lines (221) and the second segmenting lines (223), and
wherein both a radial strain and a circumference strain are calculated by the physical quantity calculating unit (21) by using every two adjacent measurement points (227).

9. the measurement point tracking method according to claim 8, wherein:
the region of interest (213, 215, 217, 305) is segmented by the plurality of first segmenting lines (221) along the epicardium (211) and the endocardium (209) of the cardiac muscle (207) and the plurality of second segmenting lines (223) that are orthogonal to the plurality of first segmenting lines (221); and
the intersecting points of the first segmenting lines (221) and the second segmenting lines (223) are set as the plurality of measurement points (227).

10. The measurement point tracking method according to claim 8, wherein the region of interest (217), in the case that the ultrasonic image to be displayed on the display unit (13) is a cardiac short axis view of the object (1) including the heart chamber (205) and the cardiac muscle (207) surrounding the heart chamber (205), can be set by the measurement position setting unit (17) by being segmented into plural partial regions in the cardiac muscle (207) in the cardiac short axis view.

11. The measurement point tracking method according to claim 8, wherein the region of interest (213, 215) in the cardiac muscle (207) in the cardiac short axis view, in the case that the ultrasonic image to be displayed on the display unit (13) is a cardiac short axis view of the object (1) including the heart chamber (205) and the cardiac muscle (207) surrounding the heart chamber (205), can be set by the measurement position setting unit (17) by being segmented into the cardiac muscle (207) which is on the side close to the transmitting/receiving surface of the ultrasonic probe (3) and the cardiac muscle (207) which is on the side away from the transmitting/receiving surface, with the heart chamber (205) interposed therebetween.

12. The measurement point tracking method according to claim 8, wherein one of the plurality of first segmenting lines (221) or the plurality of second segmenting lines (223) that are set in the region of interest (213, 215, 217, 305) for dividing the region of interest (213, 215, 217, 305) into two regions can be set as a region-of-interest segmenting line (231), or a region-of-interest segmenting line (231) can be set on an ultrasonic image separately from the plurality of first segmenting lines (221) or the plurality of second segmenting lines (223).

13. The measurement point tracking method according to claim 8, wherein:
the ultrasonic probe (3) has configuration in which a plurality of transducers that transmit/receive ultrasonic waves to/from the object (1) are two-dimensionally arrayed or the plurality of transducers are one-dimensionally arrayed for mechanically executing spatial scanning so as to measure the reflected echo signals in plural cross sections of the tissue including the cardiac muscle (207) of the heart of the object (1);
a 3-dimensional ultrasonic image generated on the basis of the ultrasonic signal in the plural cross sections is displayed on the display unit (13); and
a 3-dimensional region of interest (305) is set by the measurement position setting unit (17) on the 3-dimensional ultrasonic image displayed on the display unit (13).

## Patentansprüche

1. Ultraschalldiagnosevorrichtung (100), die Folgendes umfasst:
eine Ultraschallsonde (3), die konfiguriert ist, Ultraschallwellen zu einer/von einer zu untersuchenden Testperson (1) zu übertragen/zu empfangen;
eine Einheit (5) zum Erzeugen eines Ultraschallsignals, die konfiguriert ist, auf der Basis des reflektierten Echosignals, das durch die Ultraschallsonde (3) in dem Querschnitt des Gewebes, der den Herzmuskel (207) des Herzens der Testperson (1) enthält, empfangen wird, ein Ultraschallsignal zu erzeugen;
eine Einheit (7) zum Erzeugen eines Ultraschallbilds, die konfiguriert ist, auf der Basis des Ultraschallsignals ein Ultraschallbild zu erzeugen;
eine Anzeigeeinheit (13), die konfiguriert ist, das Ultraschallbildanzuzeigen;
eine Einheit (17) zum Einstellen der Messposition, die konfiguriert ist, auf dem Ultraschallbild, das auf der Anzeigeeinheit (13) angezeigt wird, einen Interessenbereich (213, 215, 217, 305) im Herzmuskel (207) einzustellen;
eine Nachverfolgungsberechnungseinheit (19), die konfiguriert ist, die Bewegung des Herzmuskels (207) in mehreren Messpunkten (227) in einem vermaschten Muster im Interessenbereich (213, 215, 217, 305) nachzuverfolgen; und
eine Einheit (21) zum Berechnen einer physikalischen Größe, die konfiguriert ist, auf der Basis des Nachverfolgungsergebnisses eine bestimmte physikalische Größe zu berechnen,
wobei:
die berechnete, physikalische Größe auf der Anzeigeeinheit (13) angezeigt wird; und
die Nachverfolgungsberechnungseinheit (19) die mehreren Messpunkte (227) in einem vermaschten Muster unter Verwendung mehrerer erster Segmentierungslinien (221) entlang des Epikards (211) und des Endokards (209) des Herzmuskels (207) und mehrerer zweiter Segmentierungslinien (223), die zu den mehreren ersten Segmentierungslinien (221) senkrecht sind, nachverfolgt,
**dadurch gekennzeichnet, dass**
die mehreren Messpunkte (227) auf jeder Kreuzung der ersten Segmentierungslinien (221) und der zweiten Segmentierungslinien (223) eingestellt sind, und
wobei die Einheit (21) zum Berechnen einer physikalischen Größe unter Verwendung jedes Paars zweier benachbarter Messpunkte (227) sowohl eine radiale Belastung als auch eine Umfangsbelastung berechnet.

2. Ultraschalldiagnosevorrichtung (100) nach Anspruch 1, wobei:
die Einheit (17) zum Einstellen der Messposition konfiguriert ist, den Interessenbereich (213, 215, 217, 305) durch die mehreren ersten Segmentierungslinien (221) entlang des Epikards (211) und des Endokards (209) des Herzmuskels (207) und durch die mehreren zweiten Segmentierungslinien (223), die zu den mehreren ersten Segmentierungslinien (221) senkrecht sind, zu segmentieren; und
die Kreuzungspunkte der ersten Segmentierungslinien (221) und der zweiten Segmentierungslinien (223) als die mehreren Messpunkte (227) einzustellen.

3. Ultraschalldiagnosevorrichtung (100) nach Anspruch 1, wobei die Einheit (17) zum Einstellen der Messposition in dem Fall, in dem das auf der Anzeigeeinheit (13) anzuzeigende Ultraschallbild eine Ansicht der kurzen kardialen Achse der Testperson (1) ist, die die Herzkammer (205) und den Herzmuskel (207), der die Herzkammer (205) umgibt, enthält, konfiguriert ist, durch Segmentieren des Herzmuskels (207) in der Ansicht der kurzen kardialen Achse in mehrere Teilbereiche einen Interessenbereich (217) einstellen zu können.

4. Ultraschalldiagnosevorrichtung (100) nach Anspruch 1, wobei die Einheit (17) zum Einstellen der Messposition in dem Fall, in dem das auf der Anzeigeeinheit (13) anzuzeigende Ultraschallbild eine Ansicht der kurzen kardialen Achse der Testperson (1) ist, die die Herzkammer (205) und den Herzmuskel (207), der die Herzkammer (205) umgibt, enthält, konfiguriert ist, den Interessenbereich (213, 215) durch Trennen des Herzmuskels (207) in der Ansicht der kurzen kardialen Achse in den Herzmuskel (207), der sich auf der Seite nahe an der Übertragungs-/Empfangsfläche der Ultraschallsonde (3) befindet und in den Herzmuskel (207), der sich auf der Seite entfernt von der Übertragungs-/Empfangsfläche befindet, einstellen zu können, wobei die Herzkammer (205) dazwischen eingeschoben ist.

5. Ultraschalldiagnosevorrichtung (100) nach Anspruch 1, wobei die Einheit (17) zum Einstellen der Messposition konfiguriert ist, eine der mehreren ersten Segmentierungslinien (221) oder eine der mehreren zweiten Segmentierungslinien (223), die im Interessenbereich (213, 215, 217, 305) eingestellt sind, als eine Interessenbereichssegmentierungslinie (231) zum Unterteilen des Interessenbereichs (213, 215, 217, 305) in zwei Bereiche einstellen zu können oder die Interessenbereichssegmentierungslinie (231) auf einem Ultraschallbild getrennt von den mehreren ersten Segmentierungslinien (221) oder den mehreren zweiten Segmentierungslinien (223) einstellen zu können.

6. Ultraschalldiagnosevorrichtung (100) nach Anspruch 1, wobei:
die Ultraschallsonde (3) eine Konfiguration aufweist, in der zum mechanischen Ausführen einer räumlichen Abtastung, um die reflektierten Echosignale in mehreren Querschnitten des Gewebes zu messen, die den Herzmuskel (207) oder das Herz der Testperson (1) enthalten, mehrere Signalgeber, die Ultraschallwellen zu der/von der Testperson (1) übertragen/empfangen, zweidimensional angeordnet sind oder die mehreren Signalgeber eindimensional angeordnet sind;
die Anzeigeeinheit (13) konfiguriert ist, ein 3-dimensionales Ultraschallbild anzuzeigen, das auf der Basis der Ultraschallsignale in den mehreren Querschnitten erzeugt wird; und
die Einheit (17) zum Einstellen der Messposition auf dem 3-dimensionalen Ultraschallbild, das auf der Anzeigeeinheit (13) angezeigt wird, einen 3-dimensionalen Interessenbereich (305) einstellt.

7. Ultraschalldiagnosevorrichtung (100) nach Anspruch 1, wobei die bestimmte physikalische Größe die myokardiale Geschwindigkeit in mehreren Messpunkten (227) im Interessenbereich (213, 215, 217, 305) und/oder die Belastung des Herzmuskels (207) in den mehreren Messpunkten (227) im Interessenbereich (213, 215, 217, 305) und/oder die Fläche des Herzmuskels (207), die durch den Interessenbereich (213, 215, 217) umrandet ist, und/oder das Volumen des Herzmuskels (207), das durch einen 3-dimensionalen Interessenbereich (305) umrandet ist, ist.

8. Verfahren zum Nachverfolgen von Messpunkten, das eine Ultraschalldiagnosevorrichtung (100) verwendet, wobei das Verfahren Folgendes umfasst:
Übertragen von Ultraschallwellen auf eine zu untersuchende Testperson (1) durch eine Ultraschallsonde (3);
Empfangen der Ultraschallwellen, die von der Testperson (1) reflektiert werden;
Erzeugen eines Ultraschallsignals durch eine Einheit (5) zum Erzeugen eines Ultraschallsignals auf der Basis des reflektierten Echosignals, das durch die Ultraschallsonde (3) in dem Querschnitt des Gewebes, der den Herzmuskel (207) des Herzens der Testperson (1) enthält, empfangen wird;
Erzeugen eines Ultraschallbilds durch eine Einheit (7) zum Erzeugen eines Ultraschallbilds auf der Basis des Ultraschallsignals;
Anzeigen des Ultraschallbilds durch eine Anzeigeeinheit (13);
Einstellen eines Interessenbereichs (213, 215, 217, 305) im Herzmuskel (207) auf dem Ultraschallbild, das auf der Anzeigeeinheit (13) angezeigt wird, durch eine Einheit (17) zum Einstellen der Messposition;
Nachverfolgen der Bewegung des Herzmuskels (207) in mehreren Messpunkten (227) in einem vermaschten Muster im Interessenbereich (213, 215, 217, 305) durch eine Nachverfolgungsberechnungseinheit (19);
Berechnen einer bestimmten physikalischen Größe durch eine Einheit (21) zum Berechnen einer physikalischen Größe auf der Basis des Nachverfolgungsergebnisses; und
Anzeigen der berechneten, physikalischen Größe auf der Anzeigeeinheit (13),
wobei der Nachverfolgungsschritt das Nachverfolgen der mehreren Messpunkte (227) in einem vermaschten Muster durch die Nachverfolgungsberechnungseinheit (19) unter Verwendung mehrerer erster Segmentierungslinien (221) entlang des Epikards (211) und des Endokards (209) des Herzmuskels (207) und mehrerer zweiter Segmentierungslinien (223), die zu den mehreren ersten Segmentierungslinien (221) senkrecht sind, umfasst,
**dadurch gekennzeichnet, dass**
die mehreren Messpunkte (227) auf jeder Kreuzung der ersten Segmentierungslinien (221) und der zweiten Segmentierungslinien (223) eingestellt sind, und
wobei unter Verwendung jedes Paars zweier benachbarter Messpunkte (227) sowohl eine radiale Belastung als auch eine Umfangsbelastung von der Einheit (21) zum Berechnen einer physikalischen Größe berechnet werden.

9. Verfahren zum Nachverfolgen von Messpunkten nach Anspruch 8, wobei:
der Interessenbereich (213, 215, 217, 305) durch die mehreren ersten Segmentierungslinien (221) entlang des Epikards (211) und des Endokards (209) des Herzmuskels (207) und durch die mehreren zweiten Segmentierungslinien (223), die zu den mehreren ersten Segmentierungslinien (221) senkrecht sind, segmentiert ist; und
die Kreuzungspunkte der ersten Segmentierungslinien (221) und der zweiten Segmentierungslinien (223) als die mehreren Messpunkte (227) eingestellt sind.

10. Verfahren zum Nachverfolgen von Messpunkten nach Anspruch 8, wobei der Interessenbereich (217) in dem Fall, in dem das auf der Anzeigeeinheit (13) anzuzeigende Ultraschallbild eine Ansicht der kurzen kardialen Achse der Testperson (1) ist, die die Herzkammer (205) und den Herzmuskel (207), der die Herzkammer (205) umgibt, enthält, durch die Einheit (17) zum Einstellen der Messposition eingestellt werden kann, indem er in der Ansicht der kurzen kardialen Achse in mehrere Teilbereiche im Herzmuskel (207) segmentiert wird.

11. Verfahren zum Nachverfolgen von Messpunkten nach Anspruch 8, wobei der Interessenbereich (213, 215) im Herzmuskel (207) in der Ansicht der kurzen kardialen Achse in dem Fall, in dem das auf der Anzeigeeinheit (13) anzuzeigende Ultraschallbild eine Ansicht der kurzen kardialen Achse der Testperson (1) ist, die die Herzkammer (205) und den Herzmuskel (207), der die Herzkammer (205) umgibt, enthält, durch die Einheit (17) zum Einstellen der Messposition eingestellt werden kann, indem er in den Herzmuskel (207), der sich auf der Seite nahe an der Übertragungs-/Empfangsfläche der Ultraschallsonde (3) befindet, und in den Herzmuskel (207), der sich auf der Seite entfernt von der Übertragungs-/Empfangsfläche befindet, segmentiert wird, wobei die Herzkammer (205) dazwischen eingeschoben ist.

12. Verfahren zum Nachverfolgen von Messpunkten nach Anspruch 8, wobei eine der mehreren ersten Segmentierungslinien (221) oder eine der mehreren zweiten Segmentierungslinien (223), die im Interessenbereich (213, 215, 217, 305) zum Unterteilen des Interessenbereichs (213, 215, 217, 305) in zwei Bereiche eingestellt sind, als eine Interessenbereichssegmentierungslinie (231) eingestellt werden kann oder eine Interessenbereichssegmentierungslinie (231) auf einem Ultraschallbild getrennt von den mehreren ersten Segmentierungslinien (221) oder den mehreren zweiten Segmentierungslinien (223) eingestellt werden kann.

13. Verfahren zum Nachverfolgen von Messpunkten nach Anspruch 8, wobei:
die Ultraschallsonde (3) eine Konfiguration aufweist, in der zum mechanischen Ausführen einer räumlichen Abtastung, um die reflektierten Echosignale in mehreren Querschnitten des Gewebes zu messen, die den Herzmuskel (207) oder das Herz der Testperson (1) enthalten, mehrere Signalgeber, die Ultraschallwellen zu der/von der Testperson (1) übertragen/empfangen, zweidimensional angeordnet sind oder die mehreren Signalgeber eindimensional angeordnet sind;
ein 3-dimensionales Ultraschallbild, das auf der Basis des Ultraschallsignals in den mehreren Querschnitten erzeugt wird, auf der Anzeigeeinheit (13) angezeigt wird; und
auf dem 3-dimensionalen Ultraschallbild, das auf der Anzeigeeinheit (13) angezeigt wird, ein 3-dimensionaler Interessenbereich (305) durch die Einheit (17) zum Einstellen der Messposition eingestellt wird.

## Revendications

1. Appareil de diagnostic à ultrasons (100) comprenant :
une sonde à ultrasons (3) configurée pour émettre/recevoir des ondes ultrasonores vers/depuis un objet à examiner (1) ;
une unité de génération de signal ultrasonore (5) configurée pour générer un signal ultrasonore sur la base du signal d'écho réfléchi, qui est reçu par la sonde à ultrasons (3), dans la section transversale des tissus incluant le muscle cardiaque (207) du coeur du sujet (1) ;
une unité de génération d'image à ultrasons (7) configurée pour générer une image à ultrasons sur la base du signal ultrasonore ;
une unité d'affichage (13) configurée pour afficher l'image à ultrasons ;
une unité de fixation de position de mesure (17) configurée pour fixer une région d'intérêt (213, 215, 217, 305) dans le muscle cardiaque (207) sur l'image à ultrasons affichée sur l'unité d'affichage (13) ;
une unité de calcul de suivi (19) configurée pour suivre le mouvement du muscle cardiaque (207) dans une pluralité de points de mesure en motif grillagé (227) dans la région d'intérêt (213, 215, 217, 305) ; et
une unité de calcul de quantité physique (21) configurée pour calculer une quantité physique spécifique sur la base du résultat du suivi,
dans lequel
la quantité physique calculée est affichée sur l'unité d'affichage (13) ; et l'unité de calcul de suivi (19) suit la pluralité de points de mesure en motif grillagé (227) en utilisant une pluralité de premières lignes de segmentation (221) le long de l'épicarde (211) et de l'endocarde (209) du muscle cardiaque (207) et une pluralité de secondes lignes de segmentation (223) qui sont perpendiculaires à la pluralité de premières lignes de segmentation (221),
**caractérisé en ce que** la pluralité de points de mesure (227) sont placés sur toutes les intersections des premières lignes de segmentation (221) et des secondes lignes de segmentation (223), et dans lequel l'unité de calcul de quantité physique (21) calcule à la fois une contrainte radiale et une contrainte circonférentielle en utilisant deux points de mesure adjacents (227) à chaque fois.

2. Appareil de diagnostic à ultrasons (100) selon la revendication 1, dans lequel :
l'unité de fixation de position de mesure (17) est configurée pour segmenter la région d'intérêt (213, 215, 217, 305) par la pluralité de premières lignes de segmentation (221) le long de l'épicarde (211) et de l'endocarde (209) du muscle cardiaque (207) et la pluralité de secondes lignes de segmentation (223) qui sont orthogonales à la pluralité de premières lignes de segmentation (221) ; et
pour fixer les points d'intersection des premières lignes de segmentation (221) et des secondes lignes de segmentation (223) à titre de ladite pluralité de points de mesure (227).

3. Appareil de diagnostic à ultrasons (100) selon la revendication 1, dans lequel l'unité de fixation de position de mesure (17), dans le cas où l'image à ultrasons à afficher sur l'unité d'affichage (13) est une vue suivant le petit axe cardiaque du sujet (1) incluant la chambre cardiaque (205) et le muscle cardiaque (107) entourant la chambre cardiaque (205), est configurée pour être capable de fixer une région d'intérêt (217) en segmentant le muscle cardiaque (207), dans la vue suivant le petit axe cardiaque en une pluralité de régions partielles.

4. Appareil de diagnostic à ultrasons (100) selon la revendication 1, dans lequel l'unité de fixation de position de mesure (17), dans le cas où l'image à ultrasons à afficher sur l'unité d'affichage (13) est une vue suivant le petit axe cardiaque du sujet (1) incluant la chambre cardiaque (105) et le muscle cardiaque (207) entourant la chambre cardiaque (105), est configurée pour être capable de fixer la région d'intérêt (213, 215) en séparant le muscle cardiaque (207), dans la vue suivant le petit axe cardiaque, vers l'intérieur du muscle cardiaque (207) qui est sur le côté proche de la surface d'émission/réception de la sonde à ultrasons (3) et le muscle cardiaque (207) qui est sur le côté éloigné de la surface d'émission/réception, avec la chambre cardiaque (105) interposée entre ceux-ci.

5. Appareil de diagnostic à ultrasons (100) selon la revendication 1, dans lequel l'unité de fixation de position de mesure (17) est configurée pour être capable de fixer l'une de la pluralité de premières lignes de segmentation (221) ou l'une de la pluralité de secondes lignes de segmentation (123) placées dans la région d'intérêt (213, 215, 217, 305) à titre de ligne de segmentation de régions d'intérêt (131) pour diviser la région d'intérêt (213, 215, 217, 305) en deux régions, ou de fixer la ligne de segmentation de région d'intérêt (131) sur une image à ultrasons séparément de la pluralité de premières lignes de segmentation (221) ou de la pluralité de secondes lignes de segmentation (223).

6. Appareil de diagnostic à ultrasons (100) selon la revendication 1, dans lequel :
la sonde ultrasonore (3) a une configuration dans laquelle une pluralité de transducteurs qui émettent/reçoivent des ondes ultrasonores vers/depuis le sujet (1) sont disposés en réseau à deux dimensions, ou bien la pluralité de transducteurs sont disposés en réseau à une dimension pour exécuter mécaniquement un balayage spatial de façon à mesurer les signaux d'écho réfléchis dans une pluralité de sections transversales des tissus incluant le muscle cardiaque (207) du coeur du sujet (1) ;
l'unité d'affichage (13) est configurée pour afficher une image à ultrasons tridimensionnelle générée sur la base des signaux ultrasonores dans la pluralité de sections transversales ; et
l'unité de fixation de position de mesure (17) fixe une région d'intérêt tridimensionnelle (305) sur l'image à ultrasons tridimensionnelle affichée sur l'unité d'affichage (13).

7. Appareil de diagnostic à ultrasons (100) selon la revendication 1, dans lequel la quantité physique spécifique est au moins un paramètre parmi la vitesse du myocarde dans une pluralité de points de mesure (227) dans la région d'intérêt (213, 215, 217, 305), la contrainte du muscle cardiaque (107) dans la pluralité de points de mesure (227) dans la région d'intérêt (213, 215, 217, 305), la superficie du muscle cardiaque (207) qui est tramé par la région d'intérêt (213, 215, 217), et le volume du muscle cardiaque (207) qui est tramé par une région d'intérêt tridimensionnelle (305).

8. Procédé de suivi de point de mesure utilisant un appareil de diagnostic à ultrasons (100), ledit procédé comprenant les étapes consistant à :
émettre des ondes ultrasonores vers un sujet à examiner (1) par une sonde à ultrasons (3) ;
recevoir des ondes ultrasonores réfléchies depuis ledit sujet (1) ;
générer un signal ultrasonore par une unité de génération de signal ultrasonore (5) sur la base du signal d'écho réfléchi, qui est reçu par la sonde à ultrasons (3), dans la section transversale des tissus incluant le muscle cardiaque (207) du coeur du sujet (1) ;
générer une image à ultrasons par une unité de génération d'image à ultrasons (7) sur la base du signal ultrasonore ;
afficher l'image à ultrasons par une unité d'affichage (13) ;
fixer une région d'intérêt (113, 215, 217, 305) par une unité de fixation de position de mesure (17) dans le muscle cardiaque (107) sur l'image à ultrasons qui est affichée sur l'unité d'affichage (13) ;
suivre le mouvement du muscle cardiaque (207) dans une pluralité de points de mesure en motif grillagé (227) dans la région d'intérêt (213, 215, 217, 305) par une unité de calcul de suivi (19) ;
calculer une quantité physique spécifique par une unité de calcul de quantité physique (21) sur la base du résultat de suivi ; et
afficher la quantité physique calculée sur l'unité d'affichage (13), dans lequel l'étape de suivi comprend
le suivi de la pluralité de points de mesure en motif grillagé (227) par l'unité de calcul de suivi (19) en utilisant une pluralité de premières lignes de segmentation (221) le long de l'épicarde (211) et de l'endocarde (209) du muscle cardiaque (207) et une pluralité de secondes lignes de segmentation (223) qui sont orthogonales à la pluralité de premières lignes de segmentation (221),
**caractérisé en ce que** la pluralité de points de mesure (227) sont placés sur toutes les intersections des premières lignes de segmentation (221) et des secondes lignes de segmentation (223), et
dans lequel une contrainte radiale et une contrainte circonférentielle sont toutes les deux calculées par l'unité de calcul de quantité physique (21) en utilisant deux points de mesure adjacents (227) à chaque fois.

9. Procédé de suivi de point de mesure selon la revendication 8, dans lequel :
la région d'intérêt (213, 215, 217, 305) est segmentée par la pluralité de premières lignes de segmentation (221) le long de l'épicarde (211) et de l'endocarde (209) du muscle cardiaque (207), et par la pluralité de secondes lignes de segmentation (223) qui sont perpendiculaires à la pluralité de premières lignes de segmentation (221) ; et
les points d'intersection des premières lignes de segmentation (221) et des secondes lignes de segmentation (123) sont fixés à titre de ladite pluralité de points de mesure (227).

10. Procédé de suivi de point de mesure selon la revendication 8, dans lequel :
la région d'intérêt (217), dans le cas où l'image à ultrasons à afficher sur l'unité d'affichage (13) est une vue suivant le petit axe cardiaque du sujet (1) incluant la chambre cardiaque (200) et le muscle cardiaque (207) qui entoure la chambre cardiaque (200), peut être fixée par l'unité de fixation de position de mesure (17) en étant segmentée en une pluralité de régions partielles dans le muscle cardiaque (207) dans la vue suivant le petit axe cardiaque.

11. Procédé de suivi de point de mesure selon la revendication 8, dans lequel :
la région d'intérêt (213, 215), dans le muscle cardiaque (107) dans la vue suivant le petit axe cardiaque, dans le cas où l'image à ultrasons à afficher sur l'unité d'affichage (13) est une vue suivant le petit axe cardiaque du sujet (1) incluant la chambre cardiaque (200) et le muscle cardiaque (207) entourant la chambre cardiaque (200), peut être fixée par l'unité de fixation de position de mesure (17) en étant segmentée dans le muscle cardiaque (207) qui est sur le côté proche de la surface d'émission/réception de la sonde à ultrasons (3), et le muscle cardiaque (207) qui est sur le côté éloigné de la surface d'émission/réception, la chambre cardiaque (200) étant interposée entre eux.

12. Procédé de suivi de point de mesure selon la revendication 8,
dans lequel une de la pluralité de premières lignes de segmentation (221) ou de la pluralité de secondes lignes de segmentation (223) qui sont choisies dans la région d'intérêt (213, 215, 217, 300) pour diviser la région d'intérêt (213, 215, 217, 300) en deux régions qui peuvent être fixées comme une ligne de segmentation (231) présentant un intérêt, ou une ligne de segmentation (231) d'une région d'intérêt peut être fixée sur une image à ultrasons séparément de la pluralité de premières lignes de segmentation (221) ou de la pluralité de secondes lignes de segmentation (223).

13. Procédé de suivi de point de mesure selon la revendication 8, dans lequel :
la sonde à ultrasons (3) a une configuration dans laquelle une pluralité de transducteurs qui émettent/reçoivent des ondes ultrasonores vers/depuis le sujet (1) sont disposés dans un réseau bidimensionnel ou bien la pluralité de transducteurs sont disposés dans un réseau monodimensionnel pour exécuter mécaniquement un balayage spatial,
de manière à mesurer les signaux d'écho réfléchis dans une pluralité de sections transversales des tissus incluant le muscle cardiaque (107) du coeur du sujet (1) ;
une image à ultrasons tridimensionnelle générée sur la base du signal à ultrasons dans la pluralité de sections transversales est affichée sur l'unité d'affichage (13) ; et
une région d'intérêt tridimensionnelle (300) est fixée par l'unité de fixation de position de mesure (17) sur une image à ultrasons tridimensionnelle affichée sur l'unité d'affichage (13).
